# EUROPÄISCHE PATENTSCHRIFT

(11) **EP 2 504 785 B1**
(45) Veröffentlichungstag und Bekanntmachung des Hinweises auf die Patenterteilung: **17.04.2019**
(21) Anmeldenummer: 10787015.6
(22) Anmeldetag: 23.11.2010
(51) Int. Cl.: A61M 1/36

(54) **VERFAHREN ZUM ANPASSEN VON GRENZWERTFENSTERN, STEUERVORRICHTUNG, MEDIZINISCHE BEHANDLUNGSVORRICHTUNG UND MEDIZINISCHE ÜBERWACHUNGSVORRICHTUNG**
METHOD FOR ADAPTING LIMIT VALUE RANGES, CONTROL DEVICE, MEDICAL TREATMENT DEVICE AND MEDICAL MONITORING DEVICE
PROCÉDÉ POUR ADAPTER DES FENÊTRES DE VALEUR LIMITE, DISPOSITIF DE COMMANDE, DISPOSITIF DE TRAITEMENT MÉDICAL ET DISPOSITIF DE SURVEILLANCE MÉDICALE

(30) Priorität: 24.11.2009 DE 102009054395
(43) Veröffentlichungstag der Anmeldung: 03.10.2012
(73) Patentinhaber: Fresenius Medical Care Deutschland GmbH, 61352 Bad Homburg v.d.H. (DE)
(72) Erfinder: GRUENDKEN, Martin, 61191 Rosbach (DE); HERRMANN, Uwe, 14558 Nuthetal (DE); KIPP, Sabine, 61348 Bad Homburg (DE); NACHBAUR-STURM, Christine, 53783 Eitdorf (DE); PUSINELLI, Thomas, 63674 Altenstadt (DE)
(74) Vertreter: Bobbert & Partner Patentanwälte PartmbB
(86) Internationale Anmeldenummer: PCT/EP2010/007075
(87) Internationale Veröffentlichungsnummer: WO 2011/063924

(56) Entgegenhaltungen:
- DE-A1- 10 114 383
- DE-A1-102006 032 815

## Beschreibung

Die vorliegende Erfindung betrifft ein Verfahren zum Anpassen von Grenzwertfenstern gemäß Anspruch 1. Sie betrifft ferner eine Steuer- oder Regeleinrichtung gemäß Anspruch 7, eine medizinische Behandlungsvorrichtung gemäß Anspruch 9, eine medizinische Überwachungsvorrichtung gemäß Anspruch 11, ein digitales Speichermedium gemäß Anspruch 13, ein Computer-Programm-Produkt gemäß Anspruch 14 sowie ein Computer-Programm gemäß Anspruch 15.

Aus der Praxis sind Grenzwertfenster für überwachte medizinische Messgrößen bekannt. Verlässt die überwachte medizinische Messgröße das Grenzwertfenster, so kann-dies auf Seiten der Technik eine vorbestimmte Folge haben; beispielsweise kann ein Alarm ausgelöst werden.

Im Verlauf einer Behandlung z.B. einer medizinischen Flüssigkeit kann es aus verschiedenen Gründen erforderlich sein, Grenzwertfenster von Messgrößen anzupassen. Zu diesen Gründen kann eine Drift oder eine dauerhafte Veränderung der absoluten Lage der Messwerte bedingt durch unkritische Ursachen zählen. Ein Beispiel hierfür ist eine bloße Lageänderung eines Patienten, dessen Blut extrakorporal behandelt wird.

Aus der DE 101 14 383 A1 ist eine Blutdrucküberwachungsvorrichtung mit einem Blutdruckmeßsystem zum Erfassen des Blutdrucks zu verschiedenen Zeitpunkten bekannt.

DE 198 10 069 A1 beschreibt eine Vorrichtung mit mehreren von einer Bedienperson über eine Steuereinheit einstellbaren Betriebsparametern.

Eine Aufgabe der vorliegenden Erfindung ist es, ein weiteres Verfahren zum Anpassen von Grenzwertfenstern vorzuschlagen. Ferner sollen geeignete Vorrichtungen angegeben werden.

Diese Aufgabe der vorliegenden Erfindung wird durch ein Verfahren zum Anpassen von Grenzwertfenstern mit den Merkmalen des Anspruchs 1 gelöst.

Das erfindungsgemäße Verfahren umfasst das Anpassen eines zweiten Grenzwertfensters wenigstens einer zweiten, insbesondere medizinischen, Messgröße in Abhängigkeit von der Veränderung eines ersten Grenzwertfensters einer ersten, insbesondere medizinischen, Messgröße. Dabei erfolgt die Anpassung mittels einer dazu vorgesehenen und konfigurierten Steuer- oder Regeleinrichtung.

Vorteilhafte Weiterentwicklungen sind Gegenstand von Unteransprüchen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen.

Wenn im Folgenden von ersten und zweiten medizinischen Messwerten, Grenzwertfenstern oder dergleichen die Rede ist, so liest der Fachmann mit, dass hiervon auch dritte, vierte oder weitere medizinische Messwerte, Grenzwertfenster oder dergleichen von der Erfindung umfasst sind.

Der Begriff "medizinische Messgröße", wie er hierin verwendet wird, bezeichnet einen Parameter oder eine Kenngröße oder eine Zustandsvariable, welche(r) einen Patienten betrifft.

Eine medizinische Messgröße kann einen Parameter oder eine Kenngröße oder eine Zustandsvariable betreffen oder sein, welche(r) zum Treffen einer Aussage über einen klinischen Zustand oder eine Änderung desselben herangezogen werden soll.

Die Messgröße kann durch Erfassen oder Messen aktueller Messwerte, z.B. unter Verwenden geeigneter Erfassungs- und/oder Messeinrichtungen, wie Sensoren, ermittelt, qualifiziert oder quantifiziert werden.

Die Begriffe "erste Messgröße" und "zweite Messgröße", wie sie hierin verwendet werden, bezeichnen unterschiedliche Messgrößen, wie beispielsweise Druck und Temperatur.

Eine Messgröße kann mit einer anderen Messgröße assoziiert sein. Zum Beispiel kann eine zweite Messgröße von einer ersten Messgröße abhängen. Dies muss aber nicht der Fall sein.

Beispiele für medizinische Messgrößen im Rahmen eines Behandlungsverfahrens können physiologische Messgrößen, insbesondere Vitalparameter eines Patienten, Druck, Temperatur, Zeit und dergleichen einschließen.

Der Begriff "Patient", wie er hierin verwendet wird, bezeichnet ein Lebewesen, wie einen Menschen oder ein Tier.

Bei einem Patienten kann es sich um einen Gesunden oder einen Kranken handeln.

Der Begriff "Grenzwertfenster", wie er hierin verwendet wird, bezeichnet einen Wertebereich oder ein Werteintervall um oder für eine medizinische Messgröße. Das Grenzwertfenster kann einen Bereich an für eine Messgröße zulässigen Messwerten umfassen bzw. ein für eine Messgröße zulässiges Werteintervall angeben bzw. darstellen.

Ein für eine Messgröße innerhalb des Grenzwertfensters erfasster Messwert kann als unkritisch angesehen werden.

Im Bereich der Medizin oder Medizintechnik kann ein Grenzwertfenster beispielsweise als Eingrenzung eines nichtpathologischen Bereichs verstanden werden.

Grenzwertfenster können einen oberen Grenzwert bzw. eine obere Grenze und/oder einen unteren Grenzwert bzw. eine untere Grenze aufweisen. Sie können sich von einem unteren Grenzwert bis zu einem oberen Grenzwert erstrecken oder umgekehrt, wobei die (oberen und/oder unteren) Grenzwerte im Wertbereich enthalten sein können oder nicht.

Ein Grenzwertfenster kann ein zweiseitig oder ein nur einseitig (z.B. nur nach oben oder nur nach unten) begrenzter Bereich sein. Wo immer im Zusammenhang mit der vorliegenden Erfindung von einem Grenzwertfenster die Rede ist, kann hierunter erfindungsgemäß auch ein Grenzwert (z.B. ein oberer Grenzwert oder ein unterer Grenzwert) zu verstehen sein.

Jeder Messgröße können ein oder mehrere Grenzwertfenster zugeordnet werden.

Der Begriff "Anpassen" oder "Anpassung" eines Grenzwertfensters, wie er hierin verwendet wird, bezeichnet ein Ändern oder Nachziehen des Grenzwertebereichs einer medizinischen Messgröße in Abhängigkeit von der Veränderung einer ersten medizinischen Messgröße oder deren Grenzwertfenster.

Die Anpassung der Grenzwertfenster kann relativ zum jeweiligen aktuellen Messwert einer jeden Messgröße erfolgen.

Die Anpassung der Grenzwertfenster erfolgt vorzugsweise erst nach vorheriger Bestätigung durch einen Anwender.

Der Begriff "Steuer- oder Regeleinrichtung", wie er hierin verwendet wird, bezeichnet eine Einrichtung, welche dazu ausgelegt, konfiguriert und/oder vorgesehen ist, eine Anpassung des zweiten Grenzwertfensters in Abhängigkeit von der Änderung eines ersten Grenzwertfensters zu veranlassen bzw. auszulösen.

Die Steuer- oder Regeleinrichtung kann ausgestaltet sein, eine Automatisierung bzw. einen automatischen oder automatisierten Ablauf des erfindungsgemäßen Verfahrens zu ermöglichen.

Dies kann vorteilhaft dazu beitragen, den zur Anpassung von Grenzwertfenstern erforderlichen Aufwand zu verringern.

Die Steuer- oder Regeleinrichtung kann die Ausführung aller oder im Wesentlichen aller Verfahrensschritte veranlassen. Das erfindungsgemäße Verfahren kann im Wesentlichen oder vollständig von der Steuer- oder Regeleinrichtung ausgeführt werden. Es kann teilweise von der Steuer- oder Regeleinrichtung ausgeführt werden.

In einer bevorzugten Ausführungsform erfolgt das Anpassen des zweiten Grenzwertfensters durch Betätigen nur einer Betätigungseinrichtung zum Ansteuern der Steuer- oder Regeleinrichtung.

Die Betätigung oder Ansteuerung der Steuer- oder Regeleinrichtung kann durch Drücken, Drehen, Schalten, Kippen, Berühren usw. der Betätigungseinrichtung erfolgen.

Geeignete Betätigungseinrichtungen schließen einen Knopf, eine Taste, einen Softkey, einen Hardkey, einen Schalter, einen Regler, einen Button eines Touchpads, einen Button eines Touchscreens, einen Button, welcher mittels einer externen Eingabeeinrichtung wie einer Tastatur, einer Maus, einem Stift und dergleichen, bedien- bzw. betätigbar ist, eine Spracheingabeeinrichtung und dergleichen ein.

Die Betätigung kann vorzugsweise eine einmalige Betätigung, also ein einmaliges Drücken, Kippen, Berühren usw. sein.

Die Steuer- oder Regeleinrichtung kann von einem Anwender wie Klinikpersonal z.B. Ärzten, Pflegepersonal und dergleichen bedient oder betätigt werden. Vorzugsweise erfolgt die Anpassung der Grenzwertfenster durch eine autorisierte Person.

Die Steuer- oder Regeleinrichtung kann von einer weiteren Einrichtung mittels Signalübertragung betätigt werden.

In einer weiteren bevorzugten Ausführungsform umfasst das erfindungsgemäße Verfahren das Einstellen bzw. Festlegen einer Ausprägung wenigstens eines Grenzwertfensters einer medizinischen Messgröße.

Der Begriff "Ausprägung" eines Grenzwertfensters, wie er hierin verwendet wird, bezeichnet eine Lage, eine Erstreckung des Grenzwertfensters, eine räumliche Gestalt eines z.B. zweidimensional dargestellten Wertebereichs usw., jeweils bezogen auf einen vorgegebenen Wert der Messgröße.

Das Grenzwertfenster kann Werte oberhalb eines solchen Werts der Messgröße und/oder Werte unterhalb des Werts der Messgröße umfassen.

Das Grenzwertfenster kann symmetrisch oder asymmetrisch um einen vorgegebenen Wert der Messgröße, zum Beispiel einen Anfangswert der medizinischen Messgröße zu Beginn einer Behandlung, angeordnet sein.

Der vorgegebene Wert der Messgröße kann einen Bezugswert für das Grenzwertfenster bzw. die Ausprägung desselben darstellen.

Die Ausprägung des Grenzwertfensters kann eine relative Lage, bezogen auf einen vorgegebenen oder erfassten Grundwert der Messgröße oder einen erfassten anfänglichen Wert bzw. Anfangswert der Messgröße oder einen Erwartungswert der Messgröße oder einen anderweitig vorgegebenen oder erfassten Wert der Messgröße angeben.

Die Ausprägung des Grenzwertfensters kann relativ zu einem anfänglichen Messwert einer medizinischen Messgröße für einen Patienten individuell zu Beginn einer Behandlung festgelegt werden bzw. sein.

Alternativ kann es während der Behandlung möglich sein, die Ausprägung eines Grenzwertfensters oder mancher oder aller Grenzwertfenster zu verändern. Dies kann beispielsweise durch manuelle Einstellungen durch einen Anwender, beispielsweise durch Betätigen einer Wipptaste zum Herauf- oder Heruntersetzen einzelner Werte oder Wertebereiche, erfolgen. Auf diese Weise nachangepasste bzw. nacheingestellte Grenzwerte oder Grenzwertfenster können als Grundlage für die Anpassung des zweiten Grenzwertfensters gemäß der vorliegenden Erfindung herangezogen werden.

Die Ausprägung kann eine Breite oder Höhe des Grenzwertfensters relativ zu einem anfänglichen Messwert der Messgröße oder einem vorbestimmten Wert sein.

Der Begriff "Breite oder Höhe des Grenzwertfensters", wie er hierin verwendet wird, bezeichnet eine Erstreckung des Grenzwertfensters von einem unteren Grenzwert der medizinischen Messgröße bis zu einem oberen Grenzwert der medizinischen Messgröße.

Alternativ oder zusätzlich dazu kann die Ausprägung ein Abstand zwischen einem oberen Grenzwert und/oder zwischen einem unteren Grenzwert und dem anfänglichen Messwert der medizinischen Messgröße sein.

Die Ausprägung des Grenzwertfensters bzw. der Grenzwertfenster einer oder mehrerer Messgrößen kann, zum Beispiel vor Beginn einer Behandlung, in den Einstellungen bzw. "Settings" einer medizinischen Behandlungsvorrichtung vorgegeben bzw. festgelegt werden.

Zum Anpassen der Grenzwertfensters können die für jedes Grenzwertfenster in den Settings hinterlegten Vorgaben entnommen werden. Dies kann vorzugsweise automatisch mittels einer Steuer- oder Regeleinrichtung erfolgen.

Die Vorgaben können für alle anzupassenden Grenzwertfenster gleich sein. Die Vorgaben können sich unterscheiden.

Die Aufgabe der vorliegenden Erfindung wird ferner durch eine Steuer- oder Regeleinrichtung gemäß Anspruch 7 gelöst. Alle mit dem erfindungsgemäßen Verfahren erzielbaren Vorteile lassen sich ungeschmälert auch mit der erfindungsgemäßen Steuer- oder Regeleinrichtung erreichen.

Die Steuer- oder Regeleinrichtung der vorliegenden Erfindung ist dazu vorgesehen und konfiguriert, angesteuert zu werden, um ein zweites Grenzwertfenster wenigstens einer zweiten medizinischen Messgröße in Abhängigkeit von der Veränderung eines ersten Grenzwertfensters einer ersten medizinischen Messgröße im Falle einer solchen Veränderung anzupassen.

Die erfindungsgemäße Steuer- oder Regeleinrichtung kann dazu vorgesehen und konfiguriert sein, angesteuert zu werden, um ein Verfahren der vorliegenden Erfindung durchzuführen.

Die Steuer- oder Regeleinrichtung kann dazu vorgesehen und konfiguriert sein, durch Betätigen nur einer (einzigen) Betätigungseinrichtung durch den Anwender angesteuert zu werden.

Die Aufgabe der vorliegenden Erfindung wird ferner durch eine medizinische Behandlungsvorrichtung gemäß Anspruch 9 und/oder durch eine medizinische Überwachungsvorrichtung gemäß Anspruch 11 gelöst. Alle mit dem erfindungsgemäßen Verfahren erzielbaren Vorteile lassen sich ungeschmälert wiederum auch mit der erfindungsgemäßen medizinischen Behandlungsvorrichtung und/oder der erfindungsgemäßen medizinischen Überwachungsvorrichtung erreichen.

Die erfindungsgemäße medizinische Behandlungsvorrichtung und/oder die medizinische Überwachungsvorrichtung weisen wenigstens eine Steuer- oder Regeleinrichtung gemäß der vorliegenden Erfindung auf.

Die erfindungsgemäße medizinische Behandlungsvorrichtung und die medizinische Überwachungsvorrichtung können getrennt voneinander vorliegen und zum Einsatz kommen. Sie können jedoch auch miteinander verbunden verwendet werden. Insbesondere kann die Überwachungsvorrichtung Teil der Behandlungsvorrichtung sein. Die vorliegende Erfindung umfasst alle diese vorgenannten Möglichkeiten.

Die medizinische Behandlungsvorrichtung und/oder die medizinische Überwachungsvorrichtung können (jeweils) weitere Einrichtungen wie Mess- und/oder Erfassungseinrichtungen, Ein- und/oder Ausgabeeinrichtungen, Speichereinrichtungen und dergleichen aufweisen und/oder mit solchen Einrichtungen koppelbar sein.

Die medizinische Behandlungsvorrichtung und/oder die medizinische Überwachungsvorrichtung können insbesondere wenigstens eine Betätigungseinrichtung aufweisen. Die Betätigungseinrichtung kann dazu vorgesehen und ausgestaltet sein, ein Ansteuern der Steuer- oder Regeleinrichtung zu ermöglichen. Ein solches Ansteuern kann beispielsweise durch einmaliges Betätigen der Betätigungseinrichtung erreicht bzw. ausgelöst werden.

Das einmalige Betätigen der Betätigungseinrichtung kann sich in einer erfindungsgemäßen Ausführungsform auf einen einzigen Tastendruck beschränken.

Die erfindungsgemäße medizinische Behandlungsvorrichtung ist vorzugsweise zur extrakorporalen Blutbehandlung wie beispielsweise einer Dialyse, einer Hämofiltration, einer Hämodiafiltration, einer Apherese, einer Adsorption, einer Blutoxygenierung und dergleichen geeignet. Die erfindungsgemäße medizinische Behandlungsvorrichtung kann eine Herz-Lungen-Maschine sein.

In einer bevorzugten Ausführungsform ist die medizinische Behandlungsvorrichtung eine Blutbehandlungsvorrichtung zur extrakorporalen Blutbehandlung mittels mindestens eines der Verfahren Hämodialyse, Hämofiltration, Hämodiafiltration und/oder therapeutische Apherese.

Die erfindungsgemäße medizinische Behandlungsvorrichtung kann eine Vorrichtung zum Durchführen einer Peritonealdialyse sein.

Die medizinische Überwachungsvorrichtung kann dazu ausgelegt und vorgesehen oder konfiguriert sein, den Ablauf eines medizinischen Behandlungsverfahrens, wie beispielsweise eines der vorstehend genannten Behandlungsverfahren, zu überwachen.

Die Aufgabe der vorliegenden Erfindung wird ferner durch ein digitales Speichermedium gemäß Anspruch 13 und/oder ein Computer-Programm-Produkt gemäß Anspruch 14 und/oder ein Computer-Programm gemäß Anspruch 15 gelöst. Mit ihnen lassen sich ebenfalls alle mit dem erfindungsgemäßen Verfahren erzielbaren Vorteile ungeschmälert erzielen.

Das digitale Speichermedium, welches insbesondere eine Diskette, eine CD oder eine DVD ist, weist bevorzugt elektrisch auslesbare Steuersignale auf, die so mit einem programmierbaren Computersystem zusammenwirken können, dass die maschinellen Schritte des erfindungsgemäßen Verfahrens veranlasst werden.

Dabei können alle, einige oder manche der maschinell durchgeführten Schritte des erfindungsgemäßen Verfahrens veranlasst werden. Letzteres gilt auch für das Computer-Programm-Produkt und das Computer-Programm.

Das Computer-Programm-Produkt weist vorzugsweise einen auf einem maschinenlesbaren Träger gespeichertem Programmcode zur Veranlassung der maschinell durchführbaren Schritte des erfindungsgemäßen Verfahrens, wenn das Programm-Produkt auf einem Rechner abläuft, auf.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD und dergleichen sein.

Das Computer-Programm weist einen Programmcode zur Veranlassung der maschinell durchführbaren Schritte des erfindungsgemäßen Verfahrens auf, wenn das Programm auf einem Rechner oder Computer abläuft.

Das erfindungsgemäße Verfahren kann vorteilhaft dazu dienen, die Anpassung von Grenzwertfenstern zu erleichtern. So kann es beispielsweise vorteilhaft möglich sein, mehrere Grenzwertfenster gleichzeitig nachzuziehen.

Der Einsatz der Steuer- oder Regeleinrichtung kann vorteilhaft dazu beitragen, die bei bzw. während einer Behandlung erfassten medizinischen Messgrößen auf einfache und wenig aufwendige Weise zu aktualisieren.

Die vorliegende Erfindung kann somit vorteilhaft eine vereinfachte Bedienbarkeit und/oder Handhabung einer medizinischen Behandlungsvorrichtung ermöglichen. Somit kann es vorteilhaft möglich sein, Zeit - und damit auch Kosten - einzusparen.

Zusätzliche Vorteile sind damit verbunden, dass es weniger häufig zu Fehlalarmen kommt. Ferner besteht vorteilhaft eine geringere Wahrscheinlichkeit, eine erforderliche Anpassung eines Grenzwertfensters für eine zweite, dritte oder weitere Messgröße nicht vorzunehmen und ggf. eine unerwünschte Verfahrensroutine wie ein automatisches Abschalten einer Behandlungsfunktion zu riskieren.

Hierbei ist hervorzuheben, dass die Anpassung vorzugsweise nicht ohne Anwenderaktion erfolgt, sondern dass der Anwender durch Interaktion bestätigen oder initiieren muss, dass eine Anpassung der zweiten und weiteren Grenzwertfenster erfolgen darf. Auf diese Weise kann vorteilhaft vermieden werden, eine ggf. kritische Veränderung - vor allem einer relevanten Messgröße - zu übersehen.

Die Grenzwertfenster werden erfindungsgemäß - vorzugsweise nach Bestätigung durch den autorisierten Anwender - durch die Steuer- oder Regeleinrichtung nachgezogen. Es wird somit beispielsweise nicht erst bei Verletzen des Skalenendes oder bei Verlassen eines möglicher Weise nicht mehr gültig eingestellten zweiten Grenzwertfensters einer zweiten Messgröße ein Alarm (z.B. ein Druckalarm) ausgegeben. Die neuen Werte, Drücke, Alarmgrenzen können automatisch "durch das System hindurch" nachgezogen werden. Damit ist es möglich, dass der Anwender früher und damit korrekt über eine Verletzung eines Skalenendes in Form einer Meldung oder eines Alarms informiert wird.

In einem solchen Fall der vergleichsweise frühen Meldung bei Skalenendeverletzung bzw. Verlassen des Grenzwertfensters ist der Handlungsspielraum des Anwenders erfindungsgemäß größer als bei den bekannten Systemen. In Hinblick auf den Prä-Filter-Druck als Indikator für ein Clotting im Filter kann erfindungsgemäß beispielsweise im Falle der Überwachung von medizinischen Messgrößen bei der Blutbehandlung vorteilhaft ausreichend Zeit zum Eingreifen verbleiben. Die Behandlung muss also nicht mangels Zeit zum Handeln beendet werden; andere Maßnahmen können noch ergriffen werden.

Die vorliegende Erfindung ermöglicht somit bei ihrer entsprechenden Implementierung die Funktion eines "Frühwarnsystems", so dass der Anwender noch frühzeitig erforderliche Maßnahmen wie z.B. Wechsel von Post- auf Prä-Dilution oder Anpassung der Antikoagulation ergreifen kann, bevor dies nicht mehr möglich ist. Dadurch kann z.B. die Filterstandzeit erhöht werden. Auf diese Weise können mittels der vorliegenden Erfindung ökonomische Vorteile erzielbar sein.

Im Folgenden wird die vorliegende Erfindung beispielhaft unter Bezugnahme auf die beigefügte Zeichnung beschrieben. Es gilt:
- Fig. 1: zeigt eine schematische Übersicht einer erfindungsgemäßen medizinischen Behandlungsvorrichtung; und
- Fig. 2: zeigt einen Ablaufplan des erfindungsgemäßen Verfahrens.

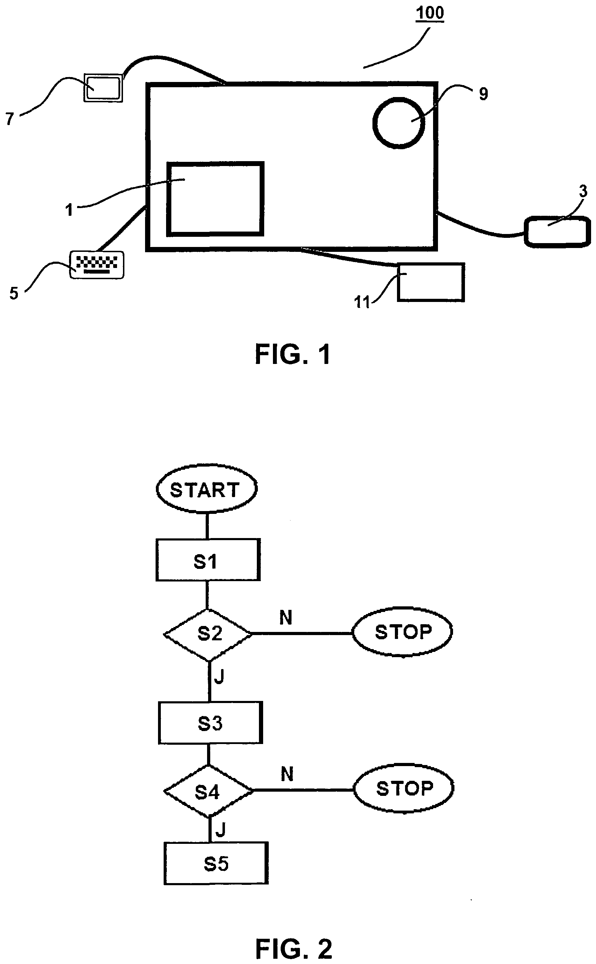

**Fig.** 1 zeigt stark schematisiert eine medizinische Behandlungsvorrichtung 100 gemäß der vorliegenden Erfindung.

Die medizinische Behandlungsvorrichtung 100 weist eine Steuer- oder Regeleinrichtung 1 auf.

Die medizinische Behandlungsvorrichtung 100 weist eine Erfassungseinrichtung 3, wie beispielsweise einen Druckmesssensor, auf.

Die medizinische Behandlungsvorrichtung 100 weist eine Eingabeeinrichtung 5, zum Beispiel eine Tastatur, sowie eine Ausgabeeinrichtung 7, zum Beispiel einen Bildschirm, auf.

Die medizinische Behandlungsvorrichtung 100 weist eine Betätigungseinrichtung 9 auf.

Die medizinische Behandlungsvorrichtung 100 weist eine Überwachungsvorrichtung 11 auf.

Die Überwachungsvorrichtung 11 kann Teil der Behandlungsvorrichtung 100 sein, wie in Fig. 1 gezeigt. Sie kann erfindungsgemäß allerdings auch losgelöst von jeder Behandlungsvorrichtung zum Einsatz kommen.

**Fig.** 2 zeigt schematisch einen Ablaufplan eines erfindungsgemäßen Verfahrens zum Anpassen eines zweiten Grenzwertfensters in Abhängigkeit der Veränderung eines ersten Grenzwertfensters.

Zum leichteren Verständnis des Zusammenspiels der einzelnen Komponenten wird auf die in Fig. 1 gezeigte Übersicht Bezug genommen. Die im erfindungsgemäßen Verfahren auftretenden Komponenten sind daher im Folgenden mit den entsprechenden, in Fig. 1 gezeigten Bezugszeichen genannt, auch wenn sie ggf. nicht auch in Fig. 2 dargestellt sind.

Zu Beginn einer Behandlung wird in einem Schritt S1 - vorzugsweise individuell für einen Patienten - eine Ausprägung der Grenzwertfenster für die während der Behandlung relevanten Messgrößen festgelegt. Die Ausprägung kann allerdings auch vorab bekannt sein und ggf. nachgeschlagen werden.

Dazu kann der Anwender die jeweilige Ausprägung des zu einer medizinischen Messgröße gehörenden Grenzwertfensters, z.B. in Form von Zahlenwerten, mittels der Eingabeeinrichtung 5 in die medizinische Behandlungsvorrichtung 100 eingeben. Die Grenzwertfenster können in den Einstellungen ("Settings") der medizinischen Behandlungsvorrichtung 100, z.B. in einer geeigneten Speichereinrichtung gespeichert werden.

Das Festlegen von Grenzwertfenstern kann ein erfindungsgemäßer Schritt sein; das Festlegen ist jedoch nicht zwingend Teil des erfindungsgemäßen Verfahrens. Es können - wie oben ausgeführt - geeignete, z.B. in den Einstellungen vorab hinterlegte Grenzwertfenster verwendet werden.

Anweisungen oder Abfragen an den Anwender können mittels der Ausgabeeinrichtung 7 dargestellt werden.

Während der Behandlung erfasst eine Mess- oder Erfassungseinrichtung 3 wenigstens eine für die Behandlung relevante Messgröße. Die Steuer- oder Regeleinrichtung 1 und/oder der Anwender können nun Ist-Werte der ersten Messgröße beobachten und daraus ableiten, ob eine Anpassung des Grenzwertfensters der ersten Messgröße sinnvoll oder erforderlich ist.

Falls eine Situation eintritt, in der eine Veränderung eines ersten Grenzwertfensters einer ersten Messgröße, zum Beispiel des venösen Drucks während einer Dialysebehandlung, erforderlich ist, kann der Anwender das erste Grenzwertfenster entsprechend verändern. Er kann dabei ggf. einem Vorschlag der Steuer- oder Regeleinrichtung durch Bestätigen folgen.

Hierzu kann in Schritt S2 zunächst eine Abfrage an den Anwender ausgegeben werden, ob der Anwender das erste Grenzwertfenster verändern möchte.

Ist dies nicht der Fall ("N" für NEIN), kann er das erfindungsgemäße Verfahren abbrechen ("STOP").

Möchte der Anwender das erste Grenzwertfenster anpassen ("J" für JA), so kann er in einem Schritt S3 eine Ausprägung des ersten Grenzwertfensters, z.B. eine Breite, einen Abstand zwischen einem oberen Grenzwert und/oder einem unteren Grenzwert und dem anfänglichen Messwert der ersten Messgröße und dergleichen, verändern.

Anschließend kann der Anwender seine Änderungen überprüfen und bestätigen. Sollten die Anpassungen nicht korrekt und/oder nicht gewünscht sein oder sich doch als nicht erforderlich herausstellen, so kann der Anwender das Grenzwertfenster auf seinen ursprünglichen Wert bzw. seine ursprüngliche Ausprägung zurücksetzen.

Hierfür können jeweils geeignete Eingabe- und/oder Bestätigungseinrichtungen vorgesehen sein.

Da vor dem Anpassen des ersten Grenzwertfensters vorzugsweise eine Bestätigung durch den Anwender erfolgt, kann vorteilhaft vermieden werden, die Grenzwertfenster unkontrolliert anzupassen und so gegebenenfalls eine kritische Veränderung der ersten Messgröße zu übersehen.

In einem Schritt S4 entscheidet der Anwender auf Anfrage hin, ob er das zweite Grenzwertfenster der zweiten Messgröße, zum Beispiel des Transmembrandrucks, und ggf. weitere Grenzwertfenster automatisch anpassen möchte.

Die zum Nachfahren bzw. Anpassen der Grenzwertfenster erforderlichen Einstellungen können in den Settings der medizinischen Behandlungsvorrichtung hinterlegt sein. Die Werte für die Grenzwertfenstergröße und/oder Grenzwertfensterlage können individuell beibehalten bleiben oder verändert werden. So kann es beispielsweise möglich sein, eine relative Lage der Grenzwertfenster zu dem als Auslöser für die Anpassung erfassten Messwert beizubehalten.

In Schritt S4 kann dem Anwender damit vorteilhaft die Möglichkeit gegeben werden, die Grenzwertfenster aller medizinischen Messgrößen, z.B. von Druckmessgrößen, gemeinsam neu zu positionieren.

Falls der Anwender die Grenzwertfenster nicht anpassen möchte ("N" für NEIN), kann das Verfahren beendet werden ("STOP").

Falls der Anwender die Grenzwertfenster anpassen möchte ("J" für JA), kann er in einem Schritt S5 durch Betätigen der Betätigungseinrichtung 9 die Steuer- oder Regeleinrichtung 1 ansteuern, um eine Anpassung des zweiten und ggf. weiterer Grenzwertfenster vorzunehmen bzw. zu veranlassen.

## Patentansprüche

1. Verfahren zum Anpassen von Grenzwertfenstern von medizinischen Messgrößen mit den Schritten:
- Verändern eines eingestellten oder festgelegten ersten Grenzwertfensters einer ersten Messgröße; und
- Anpassen eines zweiten eingestellten oder festgelegten Grenzwertfensters der zweiten Messgröße in Abhängigkeit von der Veränderung des ersten Grenzwertfensters der ersten Messgröße, wobei die Anpassung mittels einer dazu vorgesehenen und/oder konfigurierten Steuer- oder Regeleinrichtung (1) erfolgt,
**dadurch gekennzeichnet, dass** die erste und die zweite Messgröße unterschiedliche Messgrößen bezeichnen.

2. Verfahren nach Anspruch 1, wobei das Anpassen durch Betätigen nur einer Betätigungseinrichtung (9) zum Ansteuern der Steuer- oder Regeleinrichtung (1) erfolgt.

3. Verfahren nach Anspruch 1 oder 2 mit dem weiteren Schritt:
- Einstellen bzw. Festlegen einer Ausprägung wenigstens eines Grenzwertfensters einer Messgröße.

4. Verfahren nach Anspruch 3, wobei die Ausprägung des Grenzwertfensters, insbesondere relativ zu einem anfänglichen Messwert einer Messgröße, insbesondere für einen Patienten individuell, insbesondere zu Beginn einer Behandlung, festgelegt wird.

5. Verfahren nach Anspruch 4, wobei die Ausprägung eine Breite des Grenzwertfensters relativ zu einem vorbestimmten Wert oder relativ zu einem anfänglichen Messwert der Messgröße ist.

6. Verfahren nach Anspruch 4, wobei die Ausprägung ein Abstand zwischen einem oberen Grenzwert und/oder einem unteren Grenzwert einerseits, und dem vorbestimmten Wert oder dem anfänglichen Messwert der Messgröße andererseits ist.

7. Steuer- oder Regeleinrichtung (1), welche dazu vorgesehen und konfiguriert ist, angesteuert zu werden, um ein zweites Grenzwertfenster wenigstens einer zweiten Messgröße in Abhängigkeit von der Veränderung eines ersten Grenzwertfensters einer ersten Messgröße gemäß einem Verfahren der Ansprüche 1 bis 6 anzupassen.

8. Steuer- oder Regeleinrichtung (1) nach Anspruch 7, welche dazu vorgesehen und konfiguriert ist, durch einmaliges Betätigen nur einer Betätigungseinrichtung (9) durch den Anwender angesteuert zu werden.

9. Medizinische Behandlungsvorrichtung (100) mit
- wenigstens einer Steuer- oder Regeleinrichtung (1) gemäß einem der Ansprüche 7 bis 8.

10. Medizinische Behandlungsvorrichtung (100) nach Anspruch 9, ausgestaltet als Blutbehandlungsvorrichtung zur Blutbehandlung mittels mindestens eines der Verfahren Hämodialyse, Hämofiltration, Hämodiafiltration und/oder therapeutische Apherese.

11. Medizinische Überwachungsvorrichtung (11) mit
- wenigstens einer Steuer- oder Regeleinrichtung (1) gemäß einem der Ansprüche 7 bis 8.

12. Vorrichtung nach einem der Ansprüche 9 bis 11, mit wenigstens einer Betätigungseinrichtung (9), welche dazu vorgesehen und ausgestaltet ist, ein Ansteuern der Steuer- oder Regeleinrichtung (1) zu ermöglichen.

13. Digitales Speichermedium, insbesondere Diskette, CD oder DVD, mit elektrisch auslesbaren Steuersignalen, die so mit einem programmierbaren Computersystem zusammenwirken können, dass die maschinellen Schritte des Verfahrens gemäß einem der Ansprüche 1 bis 6 veranlasst werden.

14. Computer-Programm-Produkt mit auf einem maschinenlesbaren Träger gespeichertem Programmcode zur Veranlassung der maschinellen Schritte des Verfahrens gemäß einem der Ansprüche 1 bis 6, wenn das Programm-Produkt auf einem Rechner abläuft.

15. Computer-Programm mit Programmcode zur Veranlassung der maschinellen Schritte des Verfahrens gemäß einem der Ansprüche 1 bis 6, wenn das Programm auf einem Computer abläuft.

## Claims

1. A method for adapting threshold windows of medical measured variables comprising the steps of:
- changing a set or determined first threshold window of a first measured variable; and
- adjusting a second set or determined threshold window of a second measured variable depending on the change of the first threshold window of the first measured variable, wherein the adjustment is performed by means of a therefore control or regulating device (1).

2. The method according to claim 1, wherein the adjustment is performed by actuating only one actuating device (9) for driving the control or regulating device (1).

3. The method according to claim 1 or claim 2 comprising the further step of:
- setting, respectively determining a characteristic of at least one threshold window of a measured variable.

4. The method according to claim 3, wherein the characteristic of the threshold window is determined in particular relative to an initial measured value of a measured variable, in particular individually for a patient, in particular at the beginning of a treatment.

5. The method according to claim 4, wherein the characteristic is a width of the threshold window relative to a predetermined value or relative to an initial measured value of the measured variable.

6. The method according to claim 4, wherein the characteristic is a gap between an upper threshold and/or a lower threshold on the one hand, and the predetermined value or the initial measured value of the measured variable on the other hand.

7. A control or regulating device (1), which is provided and configured to be driven to adjust a second threshold window of at least one second measured variable depending on the change of a first threshold window of a first measured variable according to a method of claims 1 to 6.

8. The control or regulating device (1) according to claim 7, which is provided and configured to be driven by a user via a single actuation of only one actuating device (9).

9. A medical treatment apparatus (100) comprising:
- at least a control or regulating device (1) according to anyone of claims 7 to 8.

10. The medical treatment apparatus (100) according to claim 9, designed as a blood treatment apparatus for blood treatment by means of at least one of the methods hemodialysis, hemofiltration, hemodiafiltration and/or therapeutic apheresis.

11. A medical monitoring apparatus (11) comprising:
- at least a control or regulating device (1) according to anyone of claims 7 to 8.

12. An apparatus according to anyone of claims 9 to 11, comprising at least an actuating device (9), which is provided and configured to enable a driving of the control and regulating device (1).

13. A digital storage medium, in particular a diskette, a CD or a DVD, with electrically readable control signals, which can interact with a programmable computer system, so as to trigger the mechanical steps of the method according to anyone of claims 1 to 6.

14. A computer program product comprising a program code stored on a machine-readable medium for initiating the mechanical steps of the method according to anyone of claims 1 to 6 when the program product is running on a computer.

15. The computer program comprising a program code for triggering the mechanical steps of the method according to anyone of claims 1 to 6 when the program is running on a computer.

## Revendications

1. Un procédé destiné à adapter des fenêtres seuil de variables médicales mesurées, comprenant les étapes suivantes:
- modifier une première fenêtre seuil fixée ou déterminée d'une première variable mesurée; et
- ajuster une seconde fenêtre seuil fixée ou déterminée d'une seconde variable mesurée en fonction de la modification de la première fenêtre seuil de la première variable mesurée, où l'ajustement est effectué au moyen d'un dispositif de commande ou de régulation (1) prévu et/ou configuré pour cela.

2. Le procédé selon la première revendication, où l'ajustement est effectué en actionnant seulement un dispositif d'actionnement (9) pour commander le dispositif de commande ou de régulation (1).

3. Le procédé selon la revendication 1 ou 2 comprenant l'étape supplémentaire suivante:
- fixer, respectivement déterminer une caractéristique d'au moins une fenêtre seuil d'une variable mesurée.

4. Le procédé selon la revendication 3, où la caractéristique de la fenêtre seuil est déterminée, en particulier par rapport à une valeur mesurée initiale d'une variable mesurée, en particulier individuellement pour un patient, en particulier au début d'un traitement.

5. Le procédé selon la revendication 4, où la caractéristique est une largeur de la fenêtre seuil par rapport à une valeur mesurée prédéterminée ou par rapport à une valeur mesurée initiale d'une variable mesurée.

6. Le procédé selon la revendication 4, où la caractéristique est un intervalle entre un seuil supérieur et/ou un seuil inférieur d'une part, et la valeur prédéterminée ou la valeur mesurée initiale de la variable mesurée d'autre part.

7. Un dispositif de commande ou de régulation (1), prévu et configuré pour être actionné afin d'ajuster une seconde fenêtre seuil d'au moins une seconde variable mesurée en fonction de la modification d'une première fenêtre seuil d'une première variable mesurée selon un procédé des revendications 1 à 6.

8. Le dispositif de commande ou de régulation (1) selon la revendication 7, prévu et configuré pour être actionné par l'utilisateur via une activation unique d'un seul dispositif d'actionnement (9).

9. Un appareil de traitement médical (100) comprenant:
- au moins un dispositif de commande ou de régulation (1) selon l'une quelconque des revendications 7 à 8.

10. L'appareil de traitement médical (100) selon la revendication 9, conçu comme appareil de traitement du sang pour le traitement du sang au moyen d'au moins l'un des procédés d'hémodialyse, d'hémofiltration, d'hémodiafiltration et/ou d'aphérèse thérapeutique.

11. Un appareil de surveillance (11) comprenant
- au moins un dispositif de commande ou de régulation (1) selon l'une quelconque des revendications 7 à 8.

12. Un appareil selon l'une quelconque des revendications 9 à 11 comprenant au moins un dispositif d'actionnement (9), prévu et conçu pour permettre une commande du dispositif de commande ou de régulation (1).

13. Un support de stockage numérique, en particulier une disquette, un CD ou un DVD, comprenant des signaux de commande lisibles électriquement, pouvant interagir avec un système informatique programmable, de manière à déclencher les étapes automatisées du procédé selon l'une quelconque des revendications 1 à 6.

14. Un produit de programme d'ordinateur comprenant un code de programme stocké sur un support lisible par une machine pour déclencher les étapes automatisées du procédé selon l'une quelconque des revendications 1 à 6 lorsque le produit de programme est exécuté sur un ordinateur.

15. Le programme d'ordinateur comprenant un code de programme destiné à déclencher les étapes automatisées du procédé selon l'une quelconque des revendications 1 à 6 lorsque le programme est exécuté sur un ordinateur.
